Europäisches Patentamt

European Patent Office

Office européen des brevets

(19)

(11) Numéro de publication : **0 323 937 B1**

## (12) FASCICULE DE BREVET EUROPEEN

(45) Date de publication du fascicule du brevet :
04.12.91 Bulletin 91/49

(51) Int. Cl.⁵ : **G01N 33/00,** G01N 27/12

(21) Numéro de dépôt : 89420003.9

(22) Date de dépôt : 03.01.89

(54) **Structure de support pour capteur de gaz.**

(30) Priorité : 04.01.88 FR 8800223

(43) Date de publication de la demande :
12.07.89 Bulletin 89/28

(45) Mention de la délivrance du brevet :
04.12.91 Bulletin 91/49

(84) Etats contractants désignés :
AT BE CH DE ES GB GR IT LI LU NL SE

(56) Documents cités :
EP-A- 0 077 724
EP-A- 0 141 089
US-A- 4 457 161
PATENT ABSTRACTS OF JAPAN, vol. 7, no.
210 (P-223)[1355], 16 septembre 1983; p. 53 P
233 & JP-A-58 103 654 (MATSUSHITA DENKI
SANGYO K.K.) 20-06-1983

(73) Titulaire : **CORECI**
**4 rue Jean Desparmet**
**F-69008 Lyon (FR)**

(72) Inventeur : **Patissier, Bruno**
**F-42131 La Valla en Gier (FR)**
Inventeur : **Vignon, Gérard**
**57 cours de la République**
**F-69100 Villeurbanne (FR)**

(74) Mandataire : **Bratel, Gérard et al**
**Cabinet GERMAIN & MAUREAU B.P. 3011**
**F-69392 Lyon Cédex 03 (FR)**

## Description

La présente invention se rapporte à une structure de support pour un capteur de gaz destiné à être incorporé dans un dispositif de détection de traces de gaz dans un milieu. Plus particulièrement, cette invention concerne une structure de support, pour un capteur de gaz, se présentant sous la forme d'un petit morceau de feuille, plan et rigide, en matériau isolant électriquement (par exemple en alumine frittée, en verre, en silicium oxydé, ou en céramique plus généralement), le morceau de feuille comportant une partie centrale, qui est pleine et qui est apte à recevoir au moins, successivement et en couches superposées, une première épaisseur constituant une résistance ohmique de chauffage par effet Joule, une deuxième épaisseur en matériau électriquement isolant mais transmetteur de la chaleur, puis une troisième épaisseur qui réalise deux électrodes séparées par l'espace plan nécessaire à l'interposition d'un élément sensible entre ces deux électrodes, et comportant aussi une zone externe de connexion, réalisée par exemple sous forme de pourtour continu. Une telle structure de support est connue par le document EP-A-141089.

On connaît des capteurs de gaz en film mince qui sont constitués par un oxyde métallique, par exemple $SnO_2$ ou NiO, qui sont fabriqués à une température au plus égale à 500°C environ, cet oxyde métallique étant traité, avant mise en service, en le soumettant, sensiblement à la même température au plus égale à 500°C environ, à flux gazeux contenant un gaz activant du groupe constitué par $SO_2$ et $H_2S$.

On constate alors que la courbe de la conductance d'un tel capteur en fonction de la température présente des maxima qui sont chacun caractéristiques de la présence de traces d'un gaz déterminé dans l'air. Par exemple, elle présente à environ 100°C un maximum caractéristique de la présence d'$H_2S$, à environ 250°C un maximum caractéristique de la présence de $SO_2$, et à environ 400°C un maximum caractéristique de la présence de benzène.

Ces capteurs peuvent être réalisés soit par frittage d'un oxyde métallique, soit sous forme de couche mince ou de couche épaisse.

La présente invention a pour objet une structure d'accueil apte à recevoir indifféremment, de manière fiable, solide, et industrialisable par grandes quantités, des capteurs de gaz des trois sortes : frittés, en couche mince, ou en couche épaisse.

Par ailleurs, l'invention a pour but de limiter la température dans la zone externe de connexion, en éloignant cette zone de connexion de la partie centrale active, ceci avec le souci d'abaisser le coût de fabrication par l'utilisation de techniques de connexion plus courantes.

A cet effet, dans la structure de support pour capteur de gaz objet de la présente invention appartenant au genre indiqué en introduction, le morceau de feuille précité en matériau isolant électriquement est ajouré et comporte encore au moins deux bras reliant la partie centrale à la zone externe de connexion, dont deux bras de liaison sur lesquels sont déposées des connexion conductrices reliant les deux électrodes précitées respectivement à deux points de connexion déposés sur la zone externe de connexion, et sur lesquels sont par ailleurs déposées les liaisons et connexions associées à la résistance ohmique de chauffage.

De la sorte, cette structure est ensuite apte à recevoir indifféremment un capteur de gaz du type en couche mince, du type en couche épaisse, ou du type fritté. Les connexions situées sur la zone externe, séparées de la partie centrale, peuvent rester à une température ne dépassant pas par exemple 200°C, alors que la partie centrale est portée par exemple à 500°C ; on peut ainsi réaliser ces connexions par des techniques économiques, telles que notamment la soudure à l'étain. De plus, la partie centrale chaude peut être réalisée avec des dimensions beaucoup plus petites, donc de manière à consommer une énergie électrique moins élevée.

De toute façon, l'invention sera bien comprise, et ses avantages et autres caractéristiques ressortiront, au cours de la description suivante d'un exemple non limitatif de réalisation, en référence au dessin schématique annexé dans lequel :

— Figure 1 est une vue d'ensemble en perspective très agrandie de cette structure unitaire d'accueil pour capteur de gaz ;

— Figures 2 à 6 sont des vues en plan montrant les phases successives de fabrication de cette structure ;

— Figure 7 est une vue éclatée en coupe transversale de cette structure ;

— Figure 8 est une vue en plan partielle d'une feuille comportant un grand nombre de structures unitaires, avant découpe ; et

— Figure 9 est une vue en plan d'une variante de réalisation de cette structure.

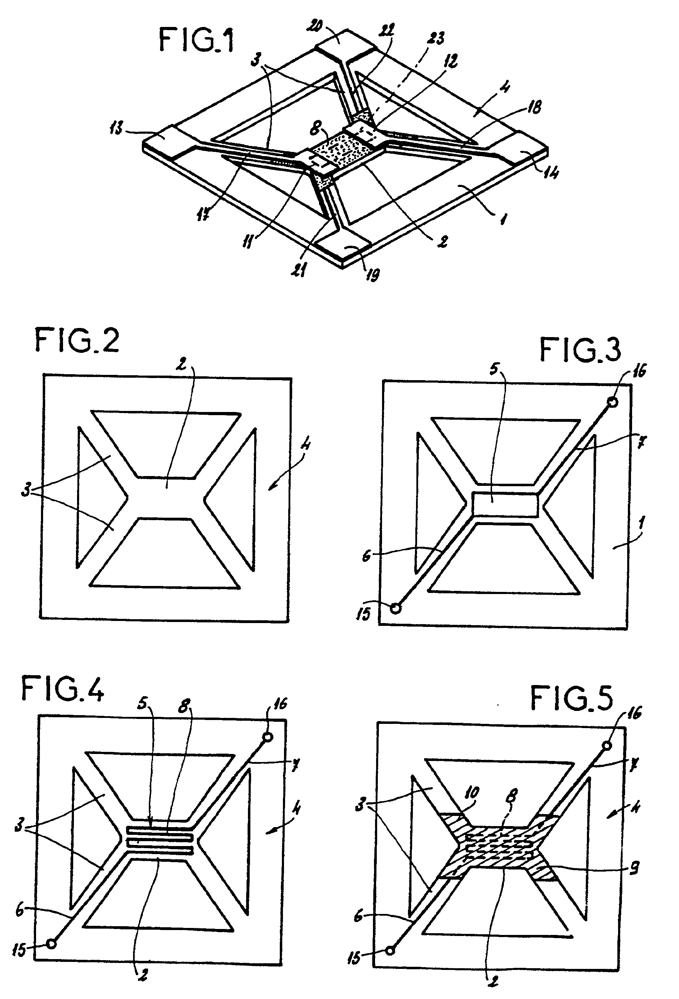

En se reportant aux figures 1 à 7, cette structure d'accueil pour capteur de gaz se présente par exemple sous la forme d'un carré 1 de 6 × 6 mm, avec un rectangle central 2 de 2 × 1 mm dont les quatre angles sont reliés aux quatre angles correspondants du carré par quatre bras 3.

Cette figure géométrique est réalisée par découpage au laser d'une embase carrée plane et rigide 4 dans une plaque d'alumine.

La figure 2 montre l'embase 4 plane et ajourée, obtenue après ce découpage. Cette embase 4 est rigide et électriquement isolante.

Sur cette embase rigide et ajourée 4 est tout d'abord, s'agissant de fabriquer la structure d'accueil de la figure 1, rapportée comme schématisé en figure 3 une figure en pâte de platine qui comporte un rectangle central 5 et deux pistes 6, 7 en forme de bras reliant deux angles opposés de ce rectangle 5 aux deux angles opposés correspondants du carré 1 que forme le pourtour de la feuille ajourée 4. Cette pâte de platine est déposée en couche par la technique connue de sérigraphie.

Le platine est un métal dont la résistance ohmique varie linéairement avec la température, de sorte que ce métal est couramment utilisé dans la technique pour faire de la mesure de température. Selon une originalité de la présente invention, on utilise ici le platine comme résistance chauffante pour le capteur de gaz.

En se reportant à la figure 4, l'étape suivante de fabrication de la structure d'accueil consiste à dessiner au laser, dans la couche rectangulaire centrale 5 en platine, une résistance linéaire 8 qui formera la résistance de chauffage du capteur à recevoir sur cette structure.

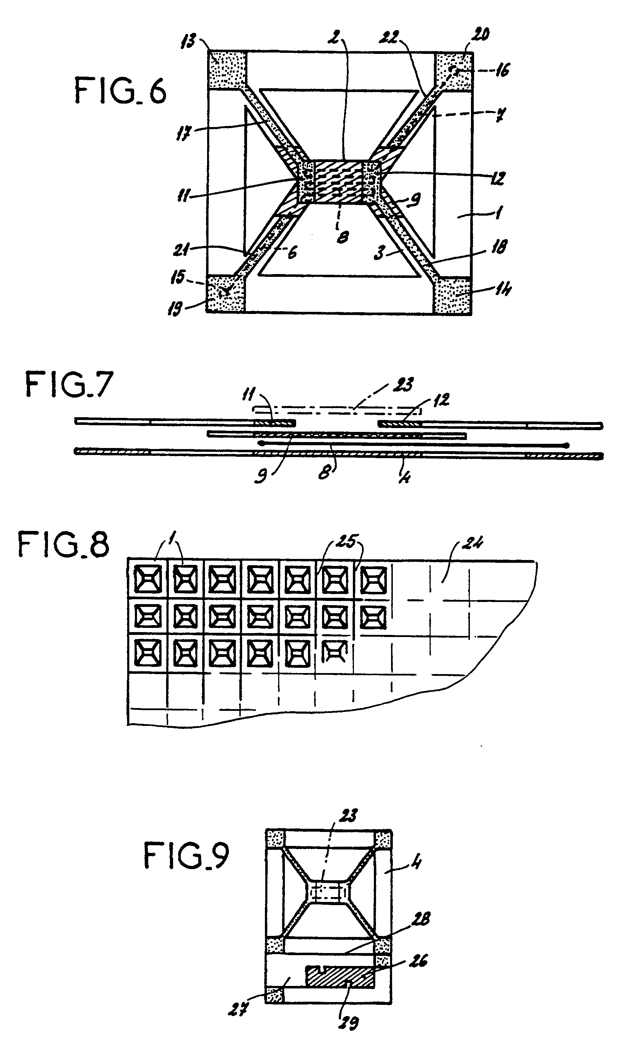

L'étape suivante de fabrication, schématisée en figure 5, consiste à déposer au centre de la structure une couche sérigraphie diélectrique 9 qui recouvre le rectangle central 2 comportant la résistance 8 en platine ainsi que les quatre amorces 10 des quatre bras 3. Cette couche diélectrique 9 permet d'isoler électriquement la résistance 8 qu'elle revêt.

La dernière phase de fabrication, schématisée en figure 6, consiste à déposer par sérigraphie, en une pâte de platine et or se prêtant à la soudure à l'étain :

— deux électrodes centrales rectangulaires 11, 12 sur les deux extrémités longitudinales du rectangle central 2, chacune de ces électrodes étant apte à recevoir une des extrémités de connexion électrique du capteur à installer sur la structure d'accueil ;

— deux plots 13, 14 de connexion placés aux deux angles diagonalement opposés du rectangle 1 autres que ceux qui reçoivent les extrémités 15, 16 des pistes de platine 6, 7, ces deux plots 13,14 étant reliés électriquement aux électrodes respectives 11 et 12 par des pistes sérigraphiées 17, 18 tracées sur les bras de liaison 3 correspondants ;

— deux autres plots 19, 20 de connexion placés sur les extrémités 15 et 16 précitées des pistes de platine 6 et 7 ; facultativement, on peut aussi recouvrir ces pistes de platine 6 et 7 par couche filiforme de pâte de platine et or 21, 22 qui s'étendrait entre les plots 19, 20 et la couche diélectrique 9 précitée.

La structure d'accueil est alors terminée et a l'aspect dessiné sur les figures 1 et 6. Elle est apte à recevoir indifféremment, entre les électrodes 11 et 12, soit un élément sensible sous forme d'un barreau fritté, tel que schématisé en traits mixtes par 23 en figure 1, soit un élément sensible réalisé en couche mince, déposé par exemple par pulvérisation cathodique, soit un élément sensible réalisé en couche épaisse, déposé par exemple par sérigraphie.

En pratique, il sera fabriqué simultanément un grand nombre de structures d'accueil telles que celle qui vient d'être décrite dans une même grande plaque d'alumine. La figure 8 montre une telle plaque 24, avec ajourages et lignes de prédécoupe 25.

Dans la plaque 24, les structures d'accueil peuvent être, après fabrication, découpées soit une par une, soit par groupes de deux, de trois, de cinq, etc...

La découpe par groupes de plusieurs structures d'accueil accolées présente des applications intéressantes. S'agissant par exemple de distinguer des traces de $SO_2$, de $H_2S$, et de benzène, on utilisera un groupe de trois structures accolées, la première structure étant chauffée à la température correspondant au pic du $SO_2$, la seconde à la température du pic du $H_2S$, et la troisième à celle du pic du benzène. Sur chaque structure, on a la faculté d'adapter le capteur optimal pour le gaz à détecter ($SO_2$, $H_2S$, ou benzène) : soit un capteur en couche mince, soit un capteur en couche épaisse, soit un capteur en matériau fritté.

Une autre possibilité est d'utiliser un assez grand nombre de structures complètes et accolées, par exemple cinq structures accolées, et de chauffer la première structure à par exemple 100°C, la seconde à 200°C,..., la cinquième à 500°C, ce qui permet de tracer de façon instantanée la courbe de la conductance en fonction de la température (les cinq capteurs étant alors tous identiques), et d'afficher sur écran ou de tracer sur papier directement le ou les pics.

La structure d'accueil ajourée peut avoir une conformation différente de celle représentée : elle peut être ronde, ovale, rectangulaire, etc..., et le nombre des bras de liaison peut être autre que quatre. Elle peut aussi être plus sophistiquée et comporter par exemple un organe de mesure de la température du capteur 23. Cet organe peut être constitué par une thermistance ou par une résistance de platine, déposées soit sur le capteur après interposition d'une mince couche diélectrique, soit au verso du rectangle central 2 et directement sur l'alumine.

Une variante particulièrement intéressante de cette structure d'accueil est schématisée figure 9. L'embase en alumine carrée 4 est prolongée, sur un des côtés du carré, par une partie rectangulaire 27 qui reçoit une résistance électrique 26 en couche épaisse. Cette résistance 26 est ajustée pour être apairée au capteur 23, le rapport de leurs résistances ohmiques étant constant, ce qui permet d'insérer cet ensemble dans un circuit électronique de mesure standard, et de remplacer si nécessaire cet ensemble par un autre sans changer quoi que ce soit à ce circuit électronique.

Entre l'embase carrée 4 et la partie rectangulaire 27 est prévue une ligne de prédécoupe 28, afin de pouvoir isoler si on le souhaite la structure portée par l'embase carrée 4.

La résistance 26 est par exemple celle aux bornes de laquelle on prend la tension de mesure, et son réglage précis est effectué de façon classique au moyen d'encoches 29.

Comme il va de soi, et comme il résulte de ce qui précède, l'invention n'est bien entendu pas limitée à l'exemple de réalisation qui vient d'être décrit. La structure pourrait ne comporter que deux bras au lieu de quatre. Elle pourrait être double, c'est-à-dire comporter sur une même embase 4, une seule et même résistance chauffante 8, et un élément sensible sur chaque face, avec électrodes d'accueil et connexions correspondantes, ces deux éléments sensibles étant identiques ou différents.

## Revendications

1. Structure de support pour capteur de gaz (23) destiné à être incorporé dans un dispositif de détection de traces de gaz dans un milieu, la structure se présentant sous la forme d'un petit morceau de feuille (4), plan et rigide, en matériau isolant électriquement, tel qu'une céramique, le morceau de feuille (4) comportant une partie centrale (2) pleine et apte à recevoir au moins, successivement et en couches superposées, une première épaisseur constituant une résistance ohmique (8) de chauffage par effet Joule, une deuxième épaisseur (9), en matériau électriquement isolant, et une troisième épaisseur réalisant deux électrodes (11, 12) séparées par l'espace plan nécessaire à l'interposition d'un élément sensible (23) entre ces deux électrodes, et comportant aussi une zone externe de connexion (1), caractérisée en ce que le morceau de feuille (4) est ajouré et comporte encore au moins deux bras (3) reliant la partie centrale (2) à la zone externe de connexion (1), dont deux bras de liaison (3) sur lesquels sont déposées des connexions conductrices (17, 18) reliant les deux électrodes précitées (11, 12) respectivement à deux points de connexion (13, 14) déposés sur la zone externe de connexion (1), et sur lesquels sont par ailleurs déposées les liaisons et connexions (19, 20) associées à la résistance ohmique de chauffage (8), de telle sorte que cette structure soit ensuite apte à recevoir indifféremment entre lesdites électrodes (11, 12) un élément sensible (23) du type couche mince, du type couche épaisse, ou du type fritté.

2. Structure de support selon la revendication 1, caractérisée en ce qu'elle comporte en outre un organe de mesure de la température du capteur (23).

3. Structure de support selon la revendication 1 ou la revendication 2, caractérisée en ce qu'elle est en outre apte à recevoir une résistance (26) apairée avec le capteur (23).

4. Structure selon la revendication 3, caractérisée en ce que la résistance apairée (26) est placée sur une partie de feuille (27) qui est séparable de celle (4) qui constitue l'embase de la structure d'accueil de l'élément sensible lui-même (23).

5. Structure selon l'une des revendications 1 à 4, caractérisée en ce qu'elle est apte à recevoir un élément sensible sur chacune de ses deux faces.

6. Structure selon la revendication 5, caractérisée en ce qu'elle comporte une seule et même résistance chauffante (8) pour les deux éléments sensibles.

## Patentansprüche

1. Trägerstruktur für Gassensoren (23), die darauf ausgelegt ist, in eine Vorrichtung zum Aufspüren von Gasspuren in einem Milieu eingesetzt zu werden, wobei die Struktur sich in Form eines kleinen ebenen und starren Blattstückes (4) präsentiert, aus einem elektrisch isolierenden Material wie einer Keramik, wobei das Blattstück (4) einen zentralen Vollabschnitt (2) aufweist, geeignet zur Aufnahme mindestens, aufeinanderfolgend und in übereinanderliegenden Schichten, einer ersten Materiallage, die einen durch den Joule-Effekt wirkenden ohmschen Heizwiderstand (8) bildet, einer zweiten Materiallage (9) aus einem elektrisch isolierenden Material und einer dritten Materiallage, die zwei Elektroden (11, 12) realisiert, die durch den flächigen Raum voneinander getrennt sind, der für die Zwischenlegung eines Spürelementes (23) zwischen diese beiden Elektroden erforderlich ist und aufweisend ferner eine externe Verbindungszone (1), **dadurch gekennzeichnet,** daß das Blattstück (4) durchbrochen ist und noch mindestens zwei Arme (3) aufweist, die den zentralen Abschnitt (2) mit der externen Verbindungszone (1) verbinden, wobei auf zwei Verbindungsarmen (39 leitende Verbindungen (17, 18) niedergelegt sind, die die beiden genannten Elektroden (11, 12) jeweils mit zwei Verbindungspunkten (13, 14) verbinden, die auf der externen Verbindungszone (1) niedergelegt sind und auf denen ferner die Bindungen und Verbindungen (19, 20) niedergelegt sind, die dem ohmschen Heizwiderstand (8) zugeordnet sind, derart, daß diese Struktur danach darauf ausgelegt ist, unterschiedslos zwischen den genannten Elektroden (11, 12) ein Spürelement (23) des Dünnschichttypes, des Dickschichttypes oder des Sintertypes aufzunehmen.

2. Trägerstruktur nach Anspruch 1, dadurch gekennzeichnet, daß sie ferner ein Temperaturmeßglied des Sensors (23) beinhaltet.

3. Trägerstruktur nach Anspruch 1 oder 2, dadurch gekennzeichnet, daß sie ferner darauf ausgelegt ist, einen an den Sensor (23) angepaßten Widerstand (26) aufzunehmen.

4. Struktur nach Anspruch 3, dadurch gekennzeichnet, daß der angepaffte Widerstand (26) auf einem Blattabschnitt (27) plaziert ist, der von demjenigen (4) abtrennbar ist, der die Grundplatte der Aufnahmestruktur des Spürelementes selbst (23) bildet.

5. Struktur nach einem der Ansprüche 1 bis 4, dadurch gekennzeichnet, daß sie darauf ausgelegt ist, ein Spürelement auf jeder ihrer beiden Seiten aufzunehmen.

6. Struktur nach Anspruch 5, dadurch gekennzeichnet, daß sie einen einzigen, für die beiden Spürelemente gemeinsamen Heizwiderstand (8) beinhaltet.

## Claims

1. A support structure for a gas sensor (23) intended to be incorporated in a device for the detection of traces of gas in an environment, the structure appearing in the form of a small piece of flat, rigid sheet (4), of electrically insulating material such as a ceramic, the piece of sheet (4) comprising a solid central portion (2) suitable to receive, successively and in superimposed layers, at least one first layer forming an electrical resistance (8) for heating by the Joule effect, a second layer (9) of electrically insulating material, and a third layer producing two electrodes (11, 12) separated by the flat space necessary for the interposition of a sensitive unit (23) between these two electrodes and also comprising an external connection zone (1), *characterized in that* the piece of sheet (4) is perforated and further comprises at least two arms (3) joining the central portion (2) to the external connection zone (1), two of which are connecting arms (3) on which conducting connections (17, 18) are deposited, joining the two above-mentioned electrodes (11, 12) to two connecting points (13, 14) deposited on the external connection zone (1), and on which are also deposited the junctions and connections (19, 20) associated with the electrical heating resistance (8), in such a way that this structure is then suitable to receive a sensitive unit (23) between the said electrodes (11, 12), no matter whether it be of the thin-layer type, of the thick-layer type, or of the sintered type.

2. A support structure according to Claim 1, *characterized in that* it additionally comprises a means of measurement of the temperature of the sensor (23).

3. A support structure according to Claim 1 or Claim 2, *characterized in that* it is also suitable to receive a resistance (26) matched to the sensor (23)

4. A structure according to Claim 3, *characterized in that* the matched resistance (26) is placed on a portion of the sheet (27) which is separable from that (4) which forms the base of the reception structure of the sensitive unit (23) itself.

5. A structure according to one of Claims 1 to 4, *characterized in that* it is suitable to receive a sensitive unit on each of its two faces.

6. A structure according to Claim 5, *characterized*

*in that* it comprises a single heating resistance (8) for the two sensitive units.

FIG.1

FIG.2

FIG.3

FIG.4

FIG.5

FIG. 6

FIG. 7

FIG. 8

FIG. 9